# EUROPEAN PATENT SPECIFICATION

(11) **EP 0 969 737 B1**
(45) Date of publication and mention of the grant of the patent: **25.06.2003**
(21) Application number: 98908575.8
(22) Date of filing: 16.02.1998
(51) Int. Cl.: A23K 1/16, A23K 1/14, A23K 1/18, A61K 45/06

(54) **PET FOOD COMPOSITION**
HAUSTIERFUTTERZUSAMMENSETZUNG
COMPOSITION ALIMENTAIRE POUR ANIMAUX DOMESTIQUES

(30) Priority: 27.03.1997 US 827405
(43) Date of publication of application: 12.01.2000
(73) Proprietor: THE IAMS COMPANY, Dayton, Ohio 45414-5801 (US)
(72) Inventor: REINHART, Gregory, Allen, Dayton, OH 45414 (US); SUNVOLD, Gregory, Dean, Eaton, OH 45320 (US)
(74) Representative: Cummings, Sean Patrick
(86) International application number: PCT/US98/03072
(87) International publication number: WO 98/043493

(56) References cited:
- EP-A- 0 674 842
- WO-A-96/03150
- US-A- 4 044 158
- US-A- 5 017 389
- US-A- 5 312 638
- WILLARD M D ET AL: "EFFECTS OF DIETARY SUPPLEMENTATION OF FRUCTO-OLIGOSACCHARIDES ON SMALL INTESTINAL BACTERIAL OVERGROWTH IN DOGS" AMERICAN JOURNAL OF VETERINARY RESEARCH, vol. 55, no. 5, May 1994, pages 654-659, XP000645762
- M.D. HOWARD ET AL.: "Effect of fermentable fiber consumption by the dog on nitrogen balance and fecal microbial nitrogen excretion" FASEB JOURNAL., vol. 10, 1996, FOR EXPERIMENTAL BIOLOGY, BETHESDA, MD US, page A257 XP002072350
- G.D. SUNVOLD ET AL.: "Dietary fiber for dogs: IV. In vitro fermentation of selected fiber sources by dog fecal inoculum and in vivo digestion and metabolism of fiber-supplemented diets" JOURNAL OF ANIMAL SCIENCE, vol. 73, no. 4, 1995, US, pages 1099-1109, XP002072351
- M. DIEZ ET AL.: "Influence of a blend of fructo-oligosaccharides and sugar beet fiber on nutrient digestibility and plasma metabolite concentrations in healthy Beagles" AMERICAN JOURNAL OF VETERINARY RESEARCH, vol. 58, no. 11, 1997, US, pages 1238-1242, XP002072352

## Description

This invention relates to a pet food composition and product containing fermentable fibers which repartitions nitrogen and increases colonic blood flow in companion animals such as dogs and cats to promote intestinal health, and improves clinical signs in animals suffering from renal disease.

Renal failure is one of the most common causes of death in dogs. In animals which suffer from renal disease, several blood indices are used to determine the severity of the disease. These indices include blood urea nitrogen (BUN) and creatinine. BUN and creatinine levels in the bloodstream increase during the course of renal failure because damage to the kidney of the animal makes the kidney inadequate to filter waste products. Because inadequate filtration of waste products is the fundamental basis of renal disease, BUN and creatinine are considered the primary indicators of renal disease.

Many dogs suffer from poor health due to renal insufficiency. It is desirable to reduce the stress placed on the kidneys of dogs with this condition. One factor that contributes to such stress is nitrogen-containing metabolites in the blood. Nitrogen in the blood is primarily removed by the kidneys. Weakened kidneys can be overworked attempting to remove nitrogen from the blood, resulting in renal failure. Therefore, a need exists for a method of reducing the amount of nitrogen in the blood of dogs without using the kidneys.

In extreme cases, medical devices such as dialysis machines can be used to remove nitrogen in the blood. However, in the case of dogs this approach is usually cost prohibitive. Also, most cases of renal insufficiency are less extreme, therefore, less invasive techniques are desirable. Particularly desirable are low cost techniques that are easy to administer.

Some research has been done in the area of nitrogen repartitioning. This involves using bodily methods of waste removal other than the kidneys to eliminate nitrogen from the blood. Younes et al., FASEB J. 8:A186 (1994) (Abstract) have experimented with using a fermentable fiber-containing diet to increase urea disposal in the rat cecum. However, those experiments were limited to the rat which has a different metabolism than the dog. For example, the dog has a nonsacculated intestine as opposed to the sacculated intestine in the rat; the dog has a nonfunctional cecum as opposed to a functional cecum in the rat; and the dog does not practice coprophagy as does the rat.

Other clinical parameters which are important to the animal suffering from renal disease are phosphorus, carbon dioxide, and triglyceride levels. Hyperphosphatemia (abnormally high blood levels of phosphorus) often manifests itself during renal disease. Previous scientific research has indicated that lowering dietary intake of phosphorus is beneficial to lessening the progression of renal disease. However, prior commercial pet food formulations have been unable to provide low levels of dietary phosphorus and still meet all of the amino acid requirements of the canine because the phosphorus component of such diets is primarily derived from ingredients high in protein. Thus, lowering the phosphorus content of the diet required a lowering of the protein components in the diet to levels which were insufficient to supply the amino acid requirements of the animal.

Carbon dioxide levels are an indicator of the level of metabolic buffering (acid-base balance) that occurs in an animal. Metabolic acidosis becomes a problem for an animal suffering from renal disease, and high carbon dioxide levels are indicators of a lack of buffering. Another parameter which is important in animals suffering from renal disease is the blood triglyceride level. It is important in the animal suffering from renal disease as the triglyceride level is often greater than in normal animals. It would be desirable to be able to control these additional parameters in the renal patient through diet.

Current dietary therapies to reduce measured BUN, creatinine, and phosphorus levels include decreasing the amount of dietary protein to levels where amino acids are present in insufficient quantities. Decreasing the level of dietary protein decreases BUN since urea is ultimately derived from protein. However, such diets may result in other problems developing for the animal as the animal's protein needs are unmet. Accordingly, there remains a need in this art for a pet food dietary composition which will result in the lowering of BUN, creatinine, and phosphorus levels in the animal without lowering dietary protein to insufficient levels. There also remains a need for a low cost, easy to administer method of reducing the amount of nitrogen in the blood of companion animals such as dogs and cats without using the kidneys and for increasing blood flow to the colon of an animal to enhance and promote intestinal health. There also remains a need in this art for a pet food dietary composition which provides improved metabolic buffering and which lowers triglyceride levels in the renal patient.

The present invention meets those needs by providing a pet food composition which improves several important clinical indicators in the renal patient and includes adequate protein, has low phosphorus levels, improves metabolic buffering, and lowers blood triglyceride levels in the animal. The pet food composition of the present invention also repartitions nitrogen in the animal by increasing nitrogen excretion via the colon. The pet food composition of the present invention also increases blood flow to the colon, increasing the amount of nitrogen excreted. Feeding an animal a diet comprising the composition results in health benefits by reducing the strain on the kidneys of the animal and improving clinical indicators while providing improved metabolic buffering, and lowering blood triglyceride levels. In addition, the increased colonic blood flow promotes a healthy gut by keeping the colonic tissues infused.

In accordance with one aspect of the present invention, a pet food composition is provided and contains a blend of at least two fermentable fibers which have an organic matter disappearance of 15 to 60 percent when fermented by fecal bacteria for a 24 hour period, the fibers being present in amounts from about 1 to 11 weight percent of supplemental total dietary fiber. The pet is maintained on the diet for a sufficient period of time to allow the fermentable fibers to ferment in the colon of the animal. The fermentation results in an increased quantity of bacteria in the colon of the animal, resulting in nitrogen being excreted through the feces of the animal. The fermentation also provides enhanced metabolic fuel availability, in the form of short chain fatty acids such as, for example, butyrates which are utilized by canine intestinal cells. Finally, animals fed a diet containing the fermentable fibers exhibit increased colonic blood flow, with such increased blood flow providing additional metabolic fuel such as, for example, glucose, to intestinal cells. Also by maintaining the animal on the diet, the pet food composition of the present invention also acts to reduce BUN and creatinine levels in the animal.

Typically, the composition comprises from about 10 to about 32% crude protein, from about 8 to about 20% fat, from about 3 to about 25% total dietary fiber, and the fermentable fibers. Preferably, the pet food composition contains from 2 to 9 weight percent of supplemental total dietary fiber of fermentable fibers. More preferably, the pet food composition contains from 3 to 7 weight percent of supplemental total dietary fiber of fermentable fibers. Most preferably, the pet food composition contains from 4 to 7 weight percent of supplemental total dietary fiber of fermentable fibers.

Preferably, the fermentable fibers have an organic matter disappearance of 20 to 50 percent. More preferably, the fermentable fibers have an organic matter disappearance of 30 to 40 percent.

The fermentable fibers are preferably selected from the group consisting of beet pulp, gum arabic, gum talha (a form of gum arabic), psyllium, rice bran, carob bean gum, citrus pulp, pectin, fructooligosaccharides, mannanoligosaccharides and mixtures thereof or as in the appended claims. More preferably, the fermentable fibers are selected from the group consisting of beet pulp, gum arabic and fructooligosaccharides. Most preferably, the fermentable fibers are a blend of beet pulp, gum talha, and fructooligosaccharides. A preferred weight ratio of beet pulp to fructooligosaccharides in the fermentable fiber blend is from about 3:1 to 6:1, and most preferably 4:1. A preferred weight ratio of beet pulp to gum talha to fructooligosaccharide is 6:2:1:5. Beet pulp provides a source of butyrate to the intestinal cells of the animal.

Optionally, the composition may also have a phosphorus content of less than about 0.25% by weight to prevent hyperphosphatemia. The composition may also optionally contain potassium citrate as a metabolic buffering agent. Inclusion of potassium citrate has been found to ameliorate metabolic acidosis in animals with renal disease.

The low-phosphorus embodiment of the pet food composition comprises from about 10 to about 32% crude protein, from about 8 to about 20% fat, and from about 3 to about 25% total dietary fiber, with the percentage of phosphorus being less than about 0.25%, all percentages by weight. In order to maintain this low phosphorus content while still providing an adequate amount of amino acids, a combination of low phosphorus-containing protein sources are utilized. Preferred low phosphorus sources of protein for the composition include soy protein isolate and corn gluten meal. Optionally, the composition further includes L-lysine and L-tryptophan as amino acid supplements.

Preferred percentages (by weight) of the protein and amino acid components of the composition are: from about 5 to about 15% soy protein isolate, from about 0.5 to about 2.5% corn gluten meal, from about 0.01 to about 0.22% L-lysine, and from about 0.01 to about 0.22% L-tryptophan. A preferred weight ratio of soy protein isolate to corn gluten meal to L-tryptophan to L-lysine is 420:46.5:5:1.

Accordingly, it is a feature of the present invention to provide a pet food product which repartitions nitrogen in pet animals by increasing nitrogen excretion via the colon which provides a low cost, easy to administer method of reducing the amount of nitrogen in the blood of the animals without using the kidneys. It is a further feature of the present invention to provide a pet food product which increases colonic blood flow in animals to promote intestinal health. It is a further object of the present invention to provide a pet food composition which improves clinical indicators in the renal patient, improves metabolic buffering, and lowers blood triglyceride levels in the animal. These, and other features and advantages of the present invention, will become apparent from the following detailed description, the accompanying drawings, and the appended claims.

By way of example, reference is now made to the drawings in which:
Fig. 1 shows the oxidation of [1-¹⁴C] butyrate by canine intestinal cells; and
Fig. 2 shows the oxidation of [U-¹⁴C] glucose by canine intestinal cells.

The present invention uses a pet food composition containing fermentable fibers to remove nitrogen from the blood of pet animals, to enhance blood flow to colonic tissues, and to enhance metabolic fuel availability to intestinal cells. The composition also, when fed to a companion animal such as a dog or cat, results in an improvement in several clinical signs associated with renal disease. This is accomplished by adding fibers having a certain range of fermentability to an animal 's diet. These fibers are a preferred nutritional source for bacteria in the colon, providing an increase in the quantity of bacteria in the colon. These bacteria also require nitrogen to reproduce. That nitrogen is derived from urea drawn into the intestinal lumen from the portal blood. Once it is inside the intestinal lumen, the nitrogen from urea and carbon skeletons from the fibers are synthesized into bacterial protein and consequently excreted in the feces.

The composition of the present invention also acts to increase colonic blood flow. Increased colonic blood flow promotes a healthy gut by keeping the colonic tissues infused with nutrients. While not wishing to be bound by any particular theory, the mechanism by which this blood flow increase occurs is believed to be either:
1) short chain fatty acids causing a relaxation of the resistance arteries of the colon; or,
2) short chain fatty acid absorption increasing intestinal metabolic activity, which elicits increased blood flow.

Metabolic fuel availability to intestinal cells is enhanced. That is, the fermentation of the fibers in the small and large intestine of the animal produces short chain fatty acids such as, for example, butyrates which are utilized by enterocytes (cells in the small intestine) and colonocytes (cells in the large intestine). Thus, the present invention provides both a direct fuel source to exogenous cell tissues in the small intestine and colon as well as an indirect fuel source (via increased colonic blood flow) to endogenous cell tissues.

Incorporation of fermentable fiber into the diet may also have several other beneficial effects. These include reducing colonic histopathologies, increasing the weight of colonic tissue or epithelial cell proliferation, and beneficially altering the intestinal microbiota. Short chain fatty acids (SCFAs) produced by fermentation of carbohydrates are associated with the trophic effects on colonic tissue and inhibition of potentially pathogenic intestinal microbiota.

The present invention uses a pet food composition containing fermentable fibers which display certain organic matter disappearance percentages. The fermentable fibers used in the present invention have an organic matter disappearance (OMD) of from about 15 to 60 percent when fermented by fecal bacteria in vitro for a 24 hour period. That is, from about 15 to 60 percent of the total organic matter originally present is fermented and converted by the fecal bacteria. The organic matter disappearance of the fibers is preferably 20 to 50 percent, and most preferably is 30 to 40 percent.

Thus, in vitro OMD percentage may be calculated as follows:$\text{{1-[(OM residue - OM blank)/OM initial]} x 100,}$ where OM residue is the organic matter recovered after 24 hours of fermentation, OM blank is the organic matter recovered in corresponding blank tubes (i.e., tubes containing medium and diluted feces, but no substrate), and OM initial is that organic matter placed into the tube prior to fermentation. Additional details of the procedure are found in Sunvold et al, J. Anim. Sci. 1995, vol. 73:1099-1109.

Fermentable fibers which are useful in the present invention produce short chain fatty acids (SCFAs) within a range of from about 28 to about 85 mmol SCFA per 1000 Calories (kcals) of metabolizable energy (ME), and more preferably within a range of from about 42 to about 71 mmol SCFA per 1000 ME kcals. This equates to a composition which has a total fermentable fiber content which yields from about 100 to about 350 mmol SCFA/kg of diet.

Millimoles of SCFAs per 1000 metabolizable energy kilocalories are calculated by first calculating the total Calories of metabolizable energy (ME) in a given diet composition per kilogram of the composition. The number of grams per 1000 kcal ME may be derived from the first calculation. Then the grams, and thus millimoles, of the fermentable fiber components of the composition may be calculated.

The fermentable fiber of the present invention may be any fiber source which intestinal bacteria present in the animal can ferment to produce significant quantities of SCFAs. "Significant quantities" of SCFAs, for purposes of this invention, are amounts over 0.5 mmol of total SCFAs/gram of substrate in a 24 hour period. Preferred fibers include beet pulp, gum arabic (including gum talha), psyllium, rice bran, carob bean gum, citrus pulp, pectin, fructooligosaccharides, mannanoligosaccharides and mixtures of these fibers. More preferably, the fermentable fibers are selected from the group consisting of beet pulp, gum arabic and fructooligosaccharides. Most preferably, the fermentable fibers are a blend of beet pulp, gum talha, and fructooligosaccharides. A preferred weight ratio of beet pulp to fructooligosaccharides in the fermentable fiber blend is from about 3:1 to 6:1, and most preferably 4:1. A preferred weight ratio of beet pulp to gum talha to fructooligosaccharide is 6:2:1.5.

The fermentable fibers are used in the pet food composition in amounts from 1 to 11 weight percent of supplemental total dietary fiber, preferably from 2 to 9 weight percent, more preferably from 3 to 7 weight percent, and most preferably from 4 to 7 weight percent.

A definition of "supplemental total dietary fiber" first requires an explanation of "total dietary fiber". "Total dietary fiber" is defined as the residue of plant food which is resistant to hydrolysis by animal digestive enzymes. The main components of total dietary fiber are cellulose, hemicellulose, pectin, lignin and gums (as opposed to "crude fiber", which only contains some forms of cellulose and lignin). "Supplemental total dietary fiber" is that dietary fiber which is added to a food product above and beyond any dietary fiber naturally present in other components of the food product. Also, a "fiber source" is considered such when it consists predominantly of fiber.

Other clinical indicators which are important to the renal patient are levels of phosphorus, carbon dioxide, and triglycerides in the blood. Abnormally high levels of phosphorus often manifests itself during renal disease. One embodiment of the present invention provides a low-phosphorus content pet food composition containing from about 10 to about 32% crude protein, from about 8 to about 20% fat, and from about 3 to about 25% total dietary fiber, with the percentage of phosphorus being less than about 0.25%, all percentages by weight. Low phosphorus levels slow the progression of renal disease and manage hyperphosphatemia through diet. In order to maintain this low phosphorus content while still providing an adequate amount of amino acids in the diet, a combination of low phosphorus containing protein sources are utilized. Preferred low phosphorus sources of protein for the composition include soy protein isolate and corn gluten meal. Moderate amounts of these high quality protein sources help to maintain glomerular filtration rate, lean muscle mass, and other bodily functions. Optionally, the composition further includes L-lysine and L-tryptophan, amino acids which are necessary to an animal's good health.

Preferred percentages (by weight) of the protein and amino acid components of the composition are: from about 5 to about 15% soy protein isolate, from about 0.5 to about 2.5% corn gluten meal, from about 0.01 to about 0.22% L-lysine, and from about 0.01 to about 0.22% L-tryptophan. A preferred weight ratio of soy protein isolate to corn gluten meal to L-tryptophan to L-lysine is 420:46.5:5:1.

Carbon dioxide levels in an animal's blood gases are also indicators of disturbances in an animal's acid-base balance. Metabolic acidosis, a condition where too much acid is present in the blood, often manifests itself in an animal with renal disease. Such a condition may be managed by adding potassium citrate to the diet of the animal to improve metabolic buffeing.

The composition of the present invention also acts to lower blood serum triglycerides in an animal with renal disease. Lowering the level of blood triglycerides in an animal with renal disease is important as high triglyceride levels are often manifested with the disease. By altering the levels of fats and fatty acids in the animal's diet, triglycerides may be lowered.

The animal is fed a diet comprising from about 10 to about 32% crude protein, from about 8 to about 20% fat, all percentages by weight. The diet also includes fermentable fibers which have an organic matter disappearance of 15 to 60 percent when fermented by fecal bacteria for a 24 hour period, the fibers being present in amounts from about 1 to 11 weight percent of supplemental total dietary fiber. Because the fermentable fibers act to lower BUN and repartition nitrogenous wastes from the urine to the feces of the animal, excessive levels of dietary fat are not needed to dilute calories provided by protein in the diet. Thus, the ratio of calories from protein in the diet to calories from fat in the diet is preferably greater than about 0.40:1. Such a diet provides a much higher ratio of protein to fat calories than previous diets while lowering triglycerides levels.

The diet may also include sources of omega-6 and omega-3 fatty acids in a ratio of from about 1:1 to about 10:1 of omega-6 fatty acids to omega-3 fatty acids. Such a combination of these fatty acids increase the activity of lipoprotein lipase (LPL) in the animal. Increased LPL activity increases fatty acid oxidation and thus decreases blood triglyceride levels. The omega-6:omega-3 fatty acid ratio also results in lower intrarenal blood pressure and reduces inflammatory mediators.

In order that the invention may be more readily understood, reference is made to the following examples which are intended to illustrate the invention, but not limit the scope thereof.

### Example 1

An experiment was conducted to determine the effect that dietary fiber fermentability had on bacterial excretion in the feces and the partitioning of waste nitrogen between the urine and the feces. Twenty purpose bred female Beagles were fed nutritionally complete diets that contained one of four fiber substrates: cellulose (low fermentability), beet pulp (moderate fermentability, moderate fermentation rate), fructooligosaccharide (FOS; moderately high fermentability, rapid fermentation rate) and a blend of gum talha, beet pulp, and FOS (see Table 1). The nitrogen content of the diet, urine, and lyophilized feces was determined and nitrogen balance calculated (See Tables 2-5). Bacterial nitrogen excretion was determined by analyzing lyophilized feces for purine concentration.

Feeding fermentable fiber to dogs decreased urinary nitrogen excretion and increased fecal nitrogen excretion. The increased fecal nitrogen occurred due to an increased microbial nitrogen excretion in the feces that resulted from fermentable fiber consumption. This experiment demonstrated that diet inclusion of fermentable fiber can partition waste nitrogen from the urine to the feces.

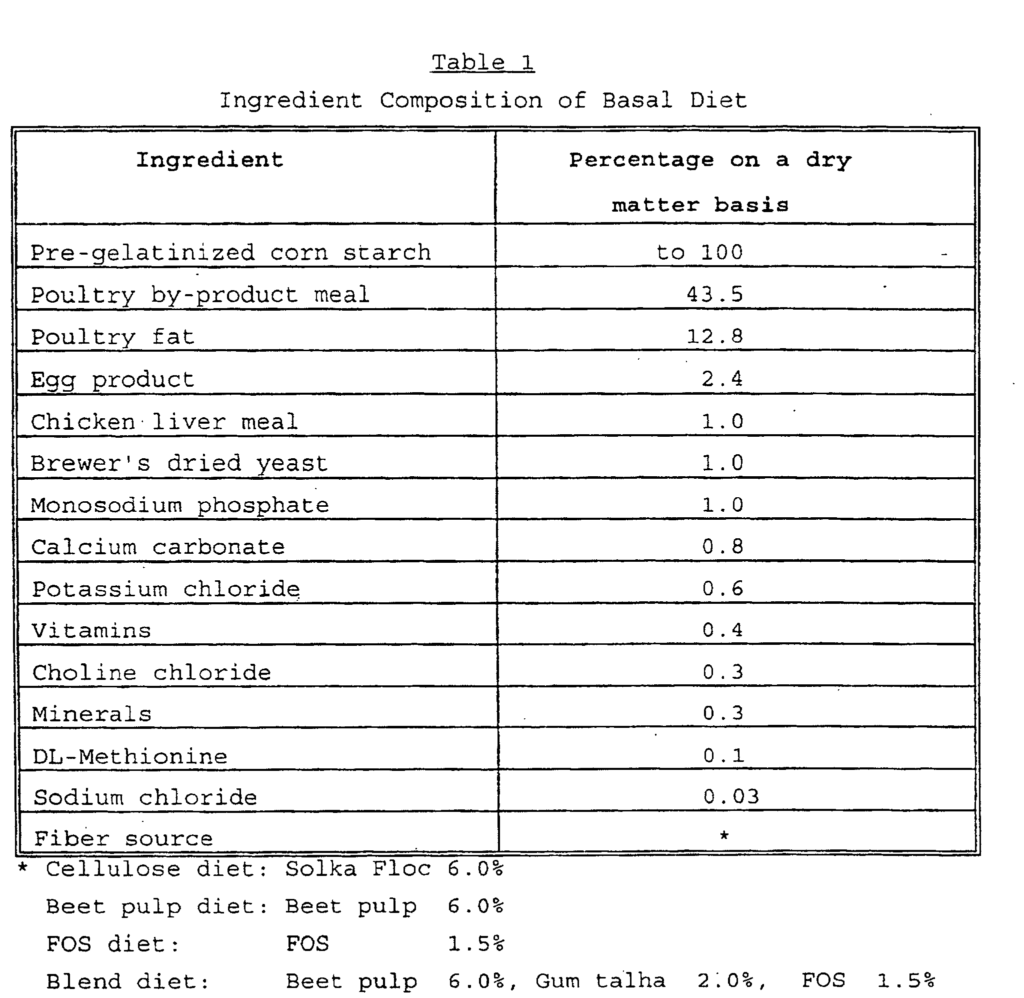

**Table 2**

| Dry Matter Intake and Digestibility of Diets Differing in Fiber Source | | | | |
|---|---|---|---|---|
| | Cellulose | Beet Pulp | FOS | Blend |
| Dry Matter Intake g/d | 258 | 224 | 195 | 225 |
| Dry Matter Digestibility % | 84 | 85 | 87 | 82 |

Intake was numerically lower and digestibility higher for the FOS diet.

**Table 3**

| Nitrogen Excretion and Digestibility of Diets Differing in Source of Fiber | | | | |
|---|---|---|---|---|
| | Cellulose | Beet Pulp | FOS | Blend |
| Nitrogen intake g/d* | 13.1 | 12.8 | 10.1 | 12.1 |
| Fecal nitrogen output g/d | 1.6 | 1.8 | 1.2 | 2.0 |
| Urinary nitrogen output g/d | 7.7 | 6.0 | 4.2 | 6.7 |
| Nitrogen balance g/d | 3.8 | 5.0 | 4.7 | 3.4 |
| Nitrogen digestibility % | 87 | 86 | 86 | 83 |
| Fecal nitrogen % of nitrogen excreted | 17.2 | 23.1 | 22.2 | 23.0 |
| Urinary nitrogen % of nitrogen excreted | 82.8 | 76.9 | 77.8 | 77.0 |

| | | | | |
|---|---|---|---|---|
| * g/d = grams per day | | | | |

Nitrogen intake tended to reflect the numerical differences observed in dry matter intake. Fecal nitrogen output was greatest for the Blend and Beet Pulp diets, intermediate for Cellulose and least for FOS diet. Urinary nitrogen output was numerically lower for dogs fed Beet Pulp, Blend, and FOS diets. Nitrogen balance was greater for Beet Pulp and FOS diets compared to Cellulose and Blend diets. Nitrogen digestibility was greatest for the Cellulose diet, intermediate for the FOS and Beet Pulp diets and lowest for the Blend diet. Fecal nitrogen expressed as a percentage of nitrogen excreted was greater for the Blend diet, FOS diet and Beet Pulp diets, as compared to the Cellulose diet. Urinary nitrogen output as a percentage of nitrogen intake was numerically greater with the Cellulose diet.

**Table 4**

| Microbial Nitrogen, and Bicinchoninic Acid (BCA) True Protein in Feces of Dogs Fed Diets Differing in Source of Fiber | | | | |
|---|---|---|---|---|
| | Cellulose | Beet Pulp | FOS | Blend |
| Microbial nitrogen g/d | 0.37 | 0.51 | 0.38 | 0.54 |
| BCA true protein g/d | 0.95 | 1.26 | 0.67 | 1.48 |
| % BcA true protein | 9.5 | 11.2 | 8.9 | 11.8 |

Microbial nitrogen output was greatest for Blend and Beet Pulp diets and lowest for FOS and Cellulose diets. Output of bicinchoninic acid (BCA) true protein was greatest for Blend, intermediate for Beet Pulp and Cellulose diets, and least for the FOS diet. BCA true protein as a percentage of fecal DM was greater for Beet Pulp and Blend diets as compared to Cellulose and FOS diets.

**Table 5**

| Blood Flow (ml/min/100g of colon) through the colic artery of dogs | | | | | |
|---|---|---|---|---|---|
| | Time | | | | x |
| | 0600 | 1200 | 1630 | 2100 | (ml/min/ 100g colon) |
| Cellulose | 12.3 | 14.8 | 10.7 | 12.0 | 12.5 |
| Beet Pulp | 13.9 | 14.1 | 12.7 | 12.2 | 13.2 |
| FOS | 22.6 | 15.0 | 11.7 | 12.4 | 15.4 |
| Blend | 17.6 | 14.8 | 13.9 | 13.4 | 14.9 |

The average blood flow to the colon was greatest with the FOS and Blend diets, intermediate with the Beet Pulp diet, and lowest with the Cellulose diet.

### Example 2

An in vitro experiment was conducted to determine the fermentability of fibrous substrates by dog fecal microflora. Feces from three female English Pointers were used as the inoculum source of anaerobic microflora. Substrates were fermented for 24 hours and then the concentrations of various short-chain fatty acids were determined. The results are shown in Table 6. The data shows that Solka Floc (a cellulose source) was essentially non-fermentable with an insignificant quantity of SCFAs being produced while lactulose was the most fermentable fiber. Fibers within the scope of the present invention, such as gum karaya, xanthan gum, gum arabic, beet pulp, gum talha, and carob bean produced moderate quantities of SCFAs, intermediate to that produced by the Solka Floc and lactulose. Also, beet pulp produced the highest quantity of butyrate, which is an important energy substrate for colonocytes.

**Table 6**

| Short-Chain Fatty Acids produced by dog fecal bacteria on various fiber substrates in a 24 hour period | | | | |
|---|---|---|---|---|
| Fiber Substrate | Short-Chain Fatty Acid (mmol/g organic matter) | | | |
| | Acetate | Propionate | Butyrate | Total SCFA |
| Solka Floc | 0.09 | 0.05 | 0.00 | 0.14 |
| Oat Fiber | 0.19 | 0.14 | 0.03 | 0.35 |
| Gum Karaya | 0.61 | 0.01 | 0.02 | 0.64 |
| Xanthan Gum | 0.80 | 0.10 | 0.05 | 0.95 |
| Gum Arabic | 0.62 | 0.47 | 0.40 | 1.49 |
| Beet Pulp | 2.03 | 0.80 | 0.70 | 3.01 |
| Gum Talha | 0.71 | 0.97 | 0.60 | 2.28 |
| Carob Bean | 2.10 | 1.44 | 0.65 | 4.19 |
| Locust Bean | 2.60 | 2.70 | 0.52 | 5.81 |
| FOS¹ | 2.86 | 2.52 | 0.30 | 5.67 |
| Pectin | 4.54 | 1.76 | 0.54 | 6.84 |
| Guar Gum | 3.07 | 3.79 | 0.41 | 7.26 |
| Lactulose | 3.47 | 4.52 | 0.35 | 8.34 |

| | | | | |
|---|---|---|---|---|
| ¹Fructooligosaccharides | | | | |

### Example 3

In vitro experiments were conducted to determine the percentage of organic matter disappearance (OMD) of fibrous substrates when exposed to dog and cat fecal microflora. Three female English Pointers provided the fecal samples for the dog anaerobic microflora. Feces from one female and two male shorthairs were used as the inoculum source of the cat anaerobic microflora. The amount of organic matter was determined for various substrates. Then, these substrates were fermented for 24 hours and the amount of organic matter remaining was determined. The results, given as the percentage of OMD, are shown in Table 7. The data shows that Solka Floc (a cellulose source) had the smallest percentage of OMD while citrus pectin had the highest. Fibers within the scope of the present invention, such as beet pulp, citrus pulp, carob bean, and gum talha had intermediate OMD percentages.

**Table 7**

| The Organic Matter Disappearance (%) of various substrates after being subjected to Dog and Cat Fecal Microflora for 24 hours | | |
|---|---|---|
| Substrate | Dog Microflora | Cat Microflora |
| Solka Floc | 4.3 | 1.2 |
| Gum Karaya | 18.5 | 27.9 |
| Xanthan Gum | 28.0 | 21.1 |
| Gum Arabic | 24.6 | 28.5 |
| Beet Pulp | 38.2 | 35.0 |
| Gum Talha | 36.3 | 35.3 |
| Citrus Pulp | 44.3 | - |
| Carob Bean Gum | 49.8 | 47.8 |
| Locust Bean Gum | 61.7 | 72.2 |
| Guar Gum | 75.3 | 74.3 |
| Citrus Pectin | 84.9 | 83.8 |

### Example 4

An in vitro experiment was conducted to determine the fermentability of selected fiber sources by dog fecal microflora. Inocula came from dogs adapted to either a non-fermentable fiber-containing diet (Solka Floc, a cellulose source) or a fermentable fiber-containing diet (citrus pulp) within the scope of the present invention.

Feces from three English Pointers adapted to each diet were used as a source of microflora to evaluate short-chain fatty acid (SCFA) production from carob bean, citrus pulp, and citrus pectin substrates. The substrates were fermented for 6 and 12 hours. The results are shown in Tables 8 and 9. The data indicates that acetate and total SCFA production was significantly greater after 6 and 12 hours of fermentation from dogs consuming the citrus pulp-containing diet, and may indicate that cellulose (Solka Floc) causes a depression in microbial activity.

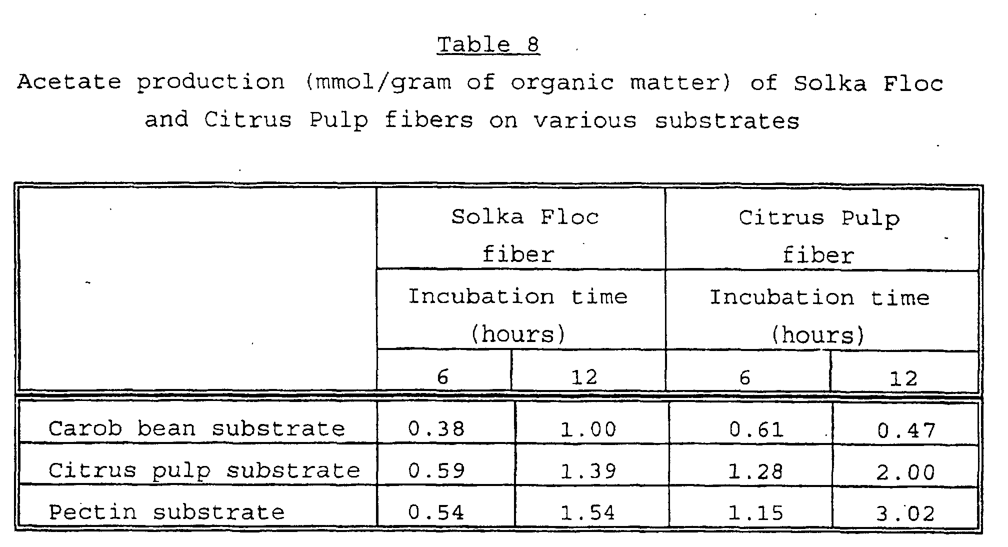

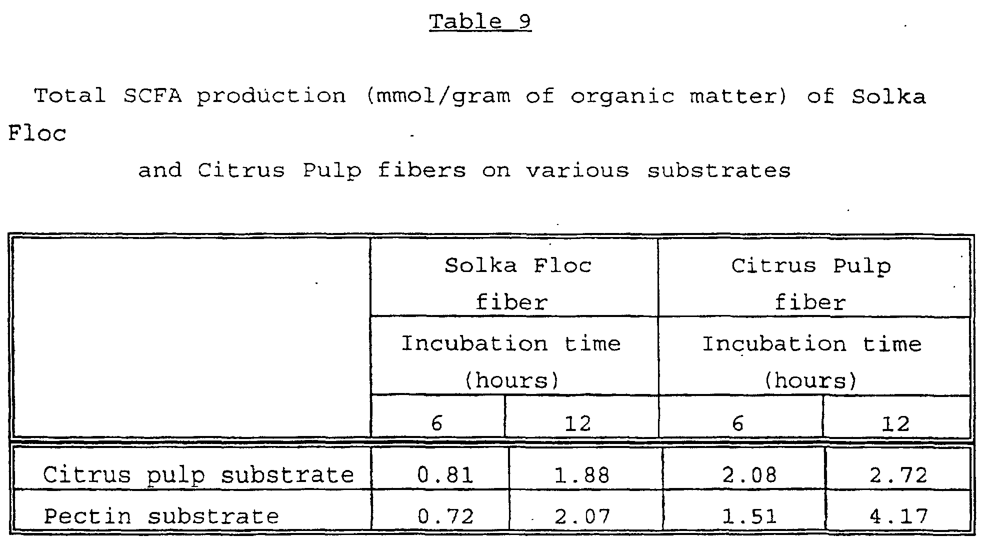

### Example 5

Enterocytes and colonocytes were isolated from the intestines of adult dogs and grown in vitro using procedures modified from Kight and Fleming, J. Nutr. Biochem. 6:27 (1995) and Marsman and McBurney, J. Nutr. 125:273 (1995). Metabolic fuel usage by the cells was determined by measuring production of ¹⁴CO₂ from radio-labeled substrates. Oxidation rates (nmoles substrate per hour per mg cell dry matter) for [1-¹⁴C]-butyrate (5 mM) [U-¹⁴C]-glucose (5mM), and L-[U-¹⁴C]-glutamine (2.5mM) were 13.5 ± 3.5, 6.5 ± .96, and 8.1 ± .21 for canine colonocytes and 18.7 ±1.7, 16.1 ± 1.0, and 32.0 ± 4.3 for canine enterocytes.

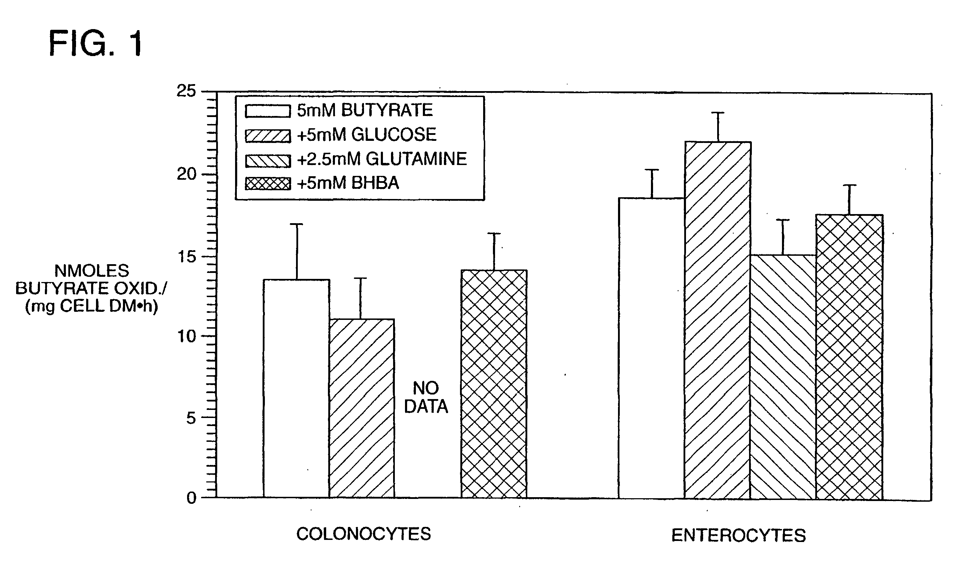

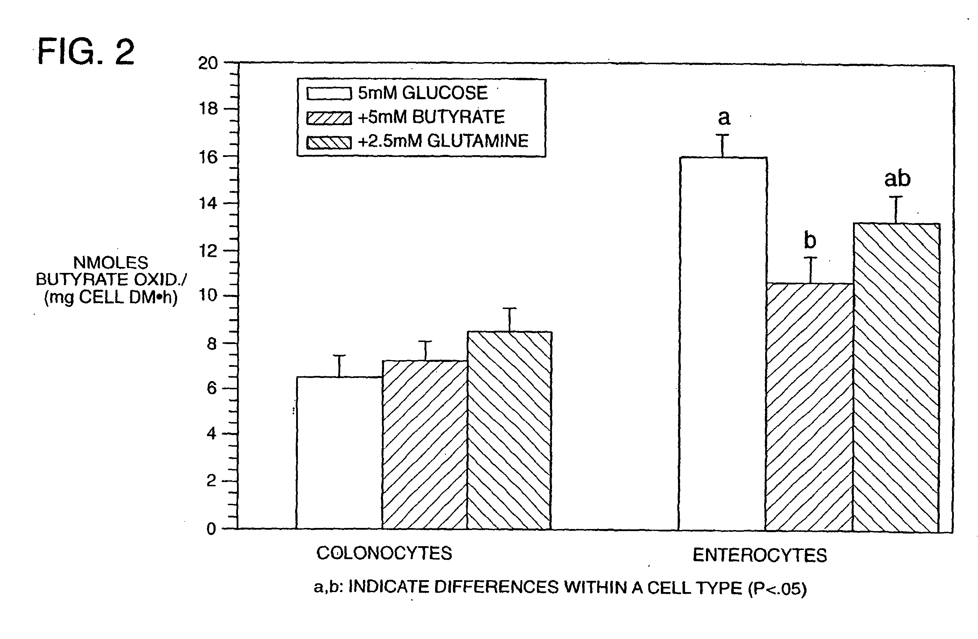

Fig. 1 shows the oxidation of [1-¹⁴C] butyrate by the canine intestinal cells. The oxidation of butyrate by colonocytes (cells in the large intestine) and enterocytes (cells in the small intestine) was not affected by medium supplementation with glucose (5mM), glutamine (2.5mM), or β-hydroxybutyric acid (BHBA) (5mM). Fig. 2 shows the oxidation of [U-¹⁴C] glucose by the canine intestinal cells. The oxidation of glucose (5mM) by colonocytes was not decreased by the addition of 2.5 mM glutamine or 5 mM β-hyroxybutyrate to the media. In contrast, the addition of butyrate decreased glucose oxidation by canine enterocytes.

From these results, canine enterocytes utilize butyrate with a greater affinity than glutamine or glucose, while canine colonocytes appear to utilize glucose and butyrate with equal affinity. The implications of these observed results indicate that feeding the dog a diet containing a source of fermentable fibers which break down into short chain fatty acids provides a source of metabolizable fuel to canine intestinal cells. Additionally, as canine intestinal cells utilize glucose as a source of fuel, the additional benefit of increased colonic blood flow resulting from a diet containing fermentable fibers enhance the availability of this important fuel source to intestinal cells.

### Example

An experiment was conducted to determine the effects on certain clinical signs in dogs with renal disease. Table 10 below summarizes the percentage of calories derived from each macronutrient in a conventional pet food diet versus the diet of the present invention. Table 11 summarizes the results obtained from four adult dogs suffering from renal disease and compare their clinical BUN, creatinine, phosphorus, carbon dioxide, and triglycerides levels before and after consuming the diet of the present invention.

**TABLE 10**

| Percentage of Calories From Each Macronutrient in Conventional Diets versus Diet of the Present Invention for Dogs With Renal Disease: | | | |
|---|---|---|---|
| Diet | Protein | Fat | Carbohydrate |
| Conventional | 10.6 | 38.7 | 50.8 |
| Formulation of the present invention | 12.9 - 17.3 | 29.4 - 31.1 | 57.7 - 51.6 |

As can be seen, the diet of the present invention provides a much greater percentage of calories from protein and less from fat than prior diets.

**TABLE 11**

| Summary of Results From Four Dogs Before and After Consuming Diet of the Present Invention: | | | | |
|---|---|---|---|---|
| | BUN | Creatinine | Phosphorus | CO₂ |
| Triglycerides | 60.3 | 3.4 | 5.9 | 19.0 |
| Before ^{a} | | | | |
| 240.0 | | | | |
| After 99.8 | 51.8 | 2.8 | 5.3 | 21.8 |
| Normal Range 6-32 | 0.4-1.1 | 2.7-5.8 | 14-30 | 31-105 |

| | | | | |
|---|---|---|---|---|
| ^{a} Prior to consuming the diet of the present invention, dogs were fed a standard commercial diet used with renal patients. | | | | |

## Claims

1. A pet food product comprising a composition containing at least two fermentable fibers, said fermentable fibers comprising a blend of beet pulp, gum talha, and fructooligosaccharides, or being a blend of beet pulp, gum arabic, and fructooligosaccharides, which fermentable fibers have an organic matter disappearance of 15 to 60 percent when fermented by fecal bacteria for a 24'hour period, said fibers being present in amounts from about 1 to 11 weight percent of supplemental total dietary fiber.

2. The pet food product of claim 1 wherein said pet food composition contains from 2 to 9 weight percent of supplemental total dietary fiber of said fermentable fibers.

3. The pet food product of claim 1 wherein said pet food composition contains from 3 to 7 weight percent of supplemental total dietary fiber of said fermentable fibers.

4. The pet food product of claim 1 wherein said pet food composition contains from 4 to 7 weight percent of supplemental total dietary fiber of said fermentable fibers.

5. The pet food product of claim 1 wherein said fermentable fibers have an organic matter disappearance of 20 to 50 percent.

6. The pet food product of claim 1 wherein said fermentable fibers have an organic matter disappearance of 30 to 40 percent.

7. The pet food product of claim 1 wherein the weight ratio of beet pulp to fructooligosaccharides is from about 3:1 to 6:1.

8. The pet food product of claim 1 wherein the weight ratio of beet pulp to gum to fructooligosaccharides is 6:2:1.5

9. The pet food product of claim 1 wherein said composition has less than about 0.25% phosphorus, all percentages by weight.

10. The pet food product of claim 1 in which said composition further comprises fermentable fibers selected from the group consisting of psyllium, rice bran, carob bean gum, citrus pulp, pectin, mannanoligosaccharides, and mixtures thereof.

11. The pet food product of claim 1 in which the total fermentable fiber content of said composition yields from about 100 to about 350 mmol SCFA/kg of diet.

12. The pet food product of claim 1 in which said composition comprises from about 10 to about 32% crude protein, from about 8 to about 20% fat, and from about 3 to about 25% total dietary fiber.

13. The pet food product of claim 12 in which said crude protein is supplied from soy protein isolate and corn gluten meal.

14. The pet food product of claim 12 in which said composition further includes L-lysine and L-tryptophan.

15. The pet food product of claim 12 in which said composition includes from about 5 to about 15% soy protein isolate, from about 0.5 to about 2.5% corn gluten meal, from about 0.01 to about 0.22% L-lysine, and from about 0.01 to about 0.22% L-tryptophan.

16. The pet food product of claim 12 in which the weight ratio of soy protein isolate to corn gluten meal to L-tryptophan to L-lysine is 420:46.5:5:1.

17. The pet food product of claim 12 further including potassium citrate.

18. The pet food product of claim 12 in which the ratio of protein calories to fat calories in said composition is greater than about 0.40:1.

## Patentansprüche

1. Haustierfutterprodukt, umfassend eine Zusammensetzung, welche wenigstens zwei fermentierbare Fasern enthält, wobei die fermentierbaren Fasern eine Mischung aus ausgelaugten Zuckerrübenschnitzeln, Talhagummi und Fructooligosacchariden umfassen oder eine Mischung aus ausgelaugten Zuckerrübenschnitzeln, Gummiarabikum und Fructooligosacchariden sind, welche fermentierbaren Fasern einen organischen Stoffabbau von 15 bis 60 Prozent aufweisen, wenn für eine Zeitspanne von 24 Stunden durch Fäkalbakterien fermentiert, wobei die Fasern in Mengen von ungefähr 1 bis 11 Gewichtsprozent zusätzlichen Gesamtnahrungsmittelfasergehalts vorhanden sind.

2. Haustierfutterprodukt nach Anspruch 1, wobei die Haustierfutterzusammensetzung von 2 bis 9 Gewichtsprozent zusätzlichen Gesamtnahrungsmittelfasergehalts der fermentierbaren Fasern enthält.

3. Haustierfutterprodukt nach Anspruch 1, wobei die Haustierfutterzusammensetzung von 3 bis 7 Gewichtsprozent zusätzlichen Gesamtnahrungsmittelfasergehalts der fermentierbaren Fasern enthält.

4. Haustierfutterprodukt nach Anspruch 1, wobei die Haustierfutterzusammensetzung von 4 bis 7 Gewichtsprozent zusätzlichen Gesamtnahrungsmittelfasergehalts der fermentierbaren Fasern enthält.

5. Haustierfutterprodukt nach Anspruch 1, wobei die fermentierbaren Fasern einen organischen Stoffabbau von 20 bis 50 Prozent aufweisen.

6. Haustierfutterprodukt nach Anspruch 1, wobei die fermentierbaren Fasern einen organischen Stoffabbau von 30 bis 40 Prozent aufweisen.

7. Haustierfutterprodukt nach Anspruch 1, wobei das Gewichtsverhältnis von ausgelaugten Zuckerrübenschnitzeln zu Fructooligosacchariden von ungefähr 3:1 bis 6:1 ist.

8. Haustierfutterprodukt nach Anspruch 1, wobei das Gewichtsverhältnis von ausgelaugten Zuckerrübenschnitzeln zu Gummi zu Fructooligosacchariden 6:2:1,5 ist.

9. Haustierfutterprodukt nach Anspruch 1, wobei die Zusammensetzung bei allen Gewichtsprozentanteilen weniger als ungefähr 0,25 % Phosphor aufweist.

10. Haustierfutterprodukt nach Anspruch 1, bei welchem die Zusammensetzung des Weiteren fermentierbare Fasern umfasst, welche aus der Gruppe bestehend aus Psyllium, Reiskleie, Johannisbrotgummi, Citruspellet, Pektin, Mannanoligosacchariden und Mischungen davon ausgewählt werden.

11. Haustierfutterprodukt nach Anspruch 1, bei welchem der Gesamtgehalt an fermentierbaren Fasern der Zusammensetzung von ungefähr 100 bis ungefähr 350 mmol SCFA/kg Nahrung ergibt.

12. Haustierfutterprodukt nach Anspruch 1, bei welchem die Zusammensetzung von ungefähr 10 bis ungefähr 32 % Rohprotein, von ungefähr 8 bis ungefähr 20 % Fett und von ungefähr 3 bis ungefähr 25 % Gesamtnahrungsmittelfasergehalt umfasst.

13. Haustierfutterprodukt nach Anspruch 12, bei welchem das Rohprotein aus Sojaproteinisolat und Maisglutenmehl geliefert wird.

14. Haustierfutterprodukt nach Anspruch 12, bei welchem die Zusammensetzung des Weiteren L-Lysin und L-Tryptophan umfasst.

15. Haustierfutterprodukt nach Anspruch 12, bei welchem die Zusammensetzung von ungefähr 5 bis ungefähr 15 % Sojaproteinisolat, von ungefähr 0,5 bis ungefähr 2,5 Maisglutenmehl, von ungefähr 0,01 bis ungefähr 0,22 % L-Lysin und von ungefähr 0,01 bis ungefähr 0,22 % L-Tryptophan umfasst.

16. Haustierfutterprodukt nach Anspruch 12, bei welchem das Gewichtsverhältnis von Sojaproteinisolat zu Maisglutenmehl zu L-Tryptophan zu L-Lysin 420:46,5:5:1 ist.

17. Haustierfutterprodukt nach Anspruch 12, welches des Weiteren Kaliumcitrat umfasst.

18. Haustierfutterprodukt nach Anspruch 12, bei welchem das Verhältnis von Proteinkalorien zu Fettkalorien in der Zusammensetzung größer als ungefähr 0,40:1 ist.

## Revendications

1. Aliment destiné aux animaux de compagnie comportant une composition contenant au moins deux types de fibres fermentescibles, lesdites fibres fermentescibles comprenant un mélange de pulpe de betterave, de gomme talha et de fructooligosaccharides ou étant un mélange de pulpe de betterave, de gomme arabique et de fructooligosaccharides, le taux de dégradation de la matière organique desdites fibres fermentescibles étant compris entre 15 et 60 pour cent à l'issue d'une fermentation d'une durée de 24 heures en présence de bactéries fécales, lesdites fibres étant présentes dans une proportion variable d'environ 1 à 11 pour cent en poids des fibres alimentaires totales apportées en supplément.

2. Aliment destiné aux animaux de compagnie selon la revendication 1, dans lequel ladite composition pour l'alimentation des animaux de compagnie contient lesdites fibres fermentescibles dans une proportion de 2 à 9 pour cent en poids des fibres alimentaires totales apportées en supplément.

3. Aliment destiné aux animaux de compagnie selon la revendication 1, dans lequel ladite composition pour l'alimentation des animaux de compagnie contient lesdites fibres fermentescibles dans une proportion de 3 à 7 pour cent en poids des fibres alimentaires totales apportées en supplément.

4. Aliment destiné aux animaux de compagnie selon la revendication 1, dans lequel ladite composition pour l'alimentation des animaux de compagnie contient lesdites fibres fermentescibles dans une proportion de 4 à 7 pour cent en poids des fibres alimentaires totales apportées en supplément.

5. Aliment destiné aux animaux de compagnie selon la revendication 1, dans lequel le taux de dégradation de la matière organique desdites fibres fermentescibles est compris entre 20 et 50 pour cent.

6. Aliment destiné aux animaux de compagnie selon la revendication 1, dans lequel le taux de dégradation de la matière organique desdites fibres fermentescibles est compris entre 30 et 40 pour cent.

7. Aliment destiné aux animaux de compagnie selon la revendication 1, dans lequel la pulpe de betterave est présente dans une proportion de 3 à 6 fois supérieure en poids à celle des fructooligosaccharides.

8. Aliment destiné aux animaux de compagnie selon la revendication 1, dans lequel pour 6 g de pulpe de betterave, on aura 2 g de gomme et 1,5 g de fructooligosaccharides.

9. Aliment destiné aux animaux de compagnie selon la revendication 1, dans lequel ladite composition contient moins de 0,25% de phosphore, tous les pourcentages étant des pourcentages en poids.

10. Aliment destiné aux animaux de compagnie selon la revendication 1, dans lequel ladite composition contient en outre des fibres fermentescibles choisies parmi le psyllium, le son de riz, la gomme de caroube, la pulpe d'agrumes, la pectine, les mannanoligosaccharides, et des mélanges de ceux-ci.

11. Aliment destiné aux animaux de compagnie selon la revendication 1, dans lequel le contenu total en fibres fermentescibles de ladite composition apporte environ 100 à 350 mmol d'acides gras à chaîne courte/kg de produit.

12. Aliment destiné aux animaux de compagnie selon la revendication 1, dans lequel ladite composition contient entre environ 10 et environ 32% de protéines brutes, entre environ 8 et environ 20% de graisses et entre environ 3 et jusqu'à environ 25% de fibres alimentaires totales.

13. Aliment destiné aux animaux de compagnie selon la revendication 12, dans lequel lesdites protéines brutes sont apportées sous la forme d'isolat de protéines de soja et de farine de gluten de maïs.

14. Aliment destiné aux animaux de compagnie selon la revendication 12, dans lequel ladite composition contient en outre de la L-lysine et du L-tryptophane.

15. Aliment destiné aux animaux de compagnie selon la revendication 12, dans lequel ladite composition contient entre environ 5 et jusqu'à environ 15% d'isolat de protéine de soja, entre environ 0,5 et jusqu'à environ 2,5% de farine de gluten de maïs, entre environ 0,01 et jusqu'à environ 0,22% de L-lysine et entre environ 0,01 et jusqu'à environ 0,22% de L-tryptophane.

16. Aliment destiné aux animaux de compagnie selon la revendication 12, dans lequel pour 420 g d'isolat de protéine de soja, on aura 46,5 g de farine de gluten de maïs, 5 g de L-tryptophane et 1 g de L-lysine.

17. Aliment destiné aux animaux de compagnie selon la revendication 12, contenant en outre du citrate de potassium.

18. Aliment destiné aux animaux de compagnie selon la revendication 12, dans lequel le rapport entre les calories apportées par les protéines et celles apportées par les graisses dans ladite composition est supérieur à environ 0,40 pour 1.
